# EUROPEAN PATENT APPLICATION

(11) **EP 4 312 225 A1**
(43) Date of publication of application: **31.01.2024**
(21) Application number: 22193278.3
(22) Date of filing: 31.08.2022
(51) Int. Cl.: G16H 30/40, G16H 40/63, G16H 40/67, G16H 80/00

(54) **COMPUTATIONAL ARCHITECTURE FOR REMOTE IMAGING EXAMINATION MONITORING TO PROVIDE ACCURATE, ROBUST AND REAL-TIME EVENTS**

(30) Priority: 27.07.2022 US 202263392550 P
(71) Applicant: Koninklijke Philips N.V., 5656 AG Eindhoven (NL)
(72) Inventor: CHADUVULA, Siva Chaitanya, Eindhoven (NL); STAROBINETS, Olga, Eindhoven (NL); TELLIS, Ranjith Naveen, 5656AG Eindhoven (NL); KOKER, Ekin, 5656AG Eindhoven (NL); DALAL, Sandeep Madhukar, 5656AG Eindhoven (NL); AMTHOR, Thomas Erik, 5656AG Eindhoven (NL); QIAN, Yuechen, 5656AG Eindhoven (NL); OXENBERG, Philip, 5656AG Eindhoven (NL)
(74) Representative: Philips Intellectual Property & Standards

(57) **Abstract**

A non-transitory computer readable medium **(26s)** stores instructions executable by at least one electronic processing device **(8, 14s)** to perform a method **(100)** of monitoring a medical imaging examination. The method includes receiving one or more video feeds **(17)** of at least an imaging bay **(3);** detecting, from the one or more video feeds, whether a medical procedure is being performed in the imaging bay; in response to the detecting indicating a medical procedure is being performed in the imaging bay, controlling a local electronic processing device **(8)** assigned to the imaging bay to process the one or more video feeds to extract and present information about the medical procedure being performed in the imaging bay; and in response to the detecting indicating a medical procedure is not being performed in the imaging bay, controlling the local electronic processing device to not process the one or more video feeds.

## Description

The following relates generally to the imaging arts, remote imaging assistance arts, remote imaging examination monitoring arts, and related arts.

### BACKGROUND OF THE INVENTION

Medical imaging, such as computed tomography (CT) imaging, magnetic resonance imaging (MRI), positron emission tomography (PET) imaging, fluoroscopy imaging, and so forth, is a critical component of providing many types of medical care, and is used in a wide range of medical fields, such as cardiology, oncology, neurology, orthopedics, to name a few. The operator of the medical imaging device used to acquire the medical images is typically a trained technologist, while interpretation of the medical images is often handled by a medical specialist such as a radiologist. Interpretation of radiology reports or findings by the radiologist, and application of those findings to the patient's specific clinical case, can be handled by the patient's general practitioner (GP) physician or a medical specialist such as a cardiologist, oncologist, orthopedic surgeon, or so forth.

Currently, diagnostic imaging is in high demand. As the world population ages, the demand for quick, safe, high quality imaging will only continue to grow, putting further pressure on imaging centers and their staff. One approach for imaging centers to boost efficiency and grow operations without concomitant increase in labor costs is through a radiology operations command center (ROCC) system. Radiology operations command centers enable teams to work across the entire network of imaging sites, providing their expertise as needed and remotely assisting less experienced technologists in carrying out high quality scans. Remote technologists or experts can monitor the local operators of scanning procedures through cameras installed in the scanning areas or from other sources, such as sensors (including radar sensors), console video feeds, microphones connected to Internet of Things (IoT) devices, and so forth. In addition, these sources can be supplemented by other data sources like Health-Level 7 (HL7) medical data feeds, medical images stored in Digital Imaging and Communications in Medicine (DICOM) format, Electronic Health Record (EHR) databases, and so forth.

ROCC enables telepresence via audio-video connectivity and provides real-time access to imaging scanner console screens and video camera feeds from scanner rooms to a remote command center. The expert users at the command center provide virtual over-the-shoulder support to the local technologists and staff conducting imaging exams. The ROCC can also provide automated assistance to the remote expert and/or local technologist. Computational algorithms on ROCC tablet hardware process real-time event data from multiple channels including console and camera and provide actionable insights to the expert technologist in real time. ROCC offers solutions for a wide range of scanning devices such as MRI, CT, X-ray, and Ultrasound and the duration of patient in a scanner room varies from a few minutes to few hours. It is important that the computations run within ROCC are run in way that these events are reliably generated in real time across different imaging modalities. Such capabilities enable expert technologists to intervene proactively based on the events and support the local technologists in their day-to-day issues.

The following discloses certain improvements to overcome these problems and others.

### SUMMARY OF THE INVENTION

In one aspect, a non-transitory computer readable medium stores instructions executable by at least one electronic processing device to perform a method of monitoring a medical imaging examination. The method includes receiving one or more video feeds of at least an imaging bay; detecting, from the one or more video feeds, whether a medical procedure is being performed in the imaging bay; in response to the detecting indicating a medical procedure is being performed in the imaging bay, controlling a local electronic processing device assigned to the imaging bay to process the one or more video feeds to extract and present information about the medical procedure being performed in the imaging bay; and in response to the detecting indicating a medical procedure is not being performed in the imaging bay, controlling the local electronic processing device to not process the one or more video feeds.

In another aspect, a support apparatus for medical imaging includes a server computer; and local electronic processing devices assigned to respective medical imaging bays and programmed to apply machine learning (ML) models to video feeds received from their respective assigned imaging bays to extract information about medical imaging procedures performed in their respective assigned imaging bays. The server computer and/or the local electronic processing devices are programmed to determine whether medical imaging procedures are being performed in the respective medical imaging bays. The server computer is programmed to perform training of the ML models including allocating ML model training tasks amongst the local electronic processing devices based on whether medical imaging procedures are being performed in the corresponding assigned medical imaging bays and receiving results of the allocated ML model training tasks from the local electronic processing devices.

In another aspect, a non-transitory computer readable medium stores instructions executable by at least one electronic processing device to perform a method of monitoring a medical imaging examination. The method includes receiving one or more video feeds of at least an imaging bay; detecting, from the one or more video feeds, whether a medical procedure is being performed in the imaging bay; in response to the detecting indicating a medical procedure is being performed in the imaging bay, controlling a local electronic processing device assigned to the imaging bay to process the one or more video feeds to extract and present information about the medical procedure being performed in the imaging bay; and in response to the detecting indicating a medical procedure is not being performed in the imaging bay, controlling the local electronic processing device to perform one or more training tasks for a machine learning (ML) component.

One advantage resides in processing images during a medical imaging examination more quickly.

Another advantage resides in conserving computing power when medical imaging examinations are not being performed.

Another advantage resides in training machine-learning (ML) models to perform imaging analysis tasks.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIG. 1 diagrammatically shows an illustrative apparatus for providing remote assistance in accordance with the present disclosure.
FIG. 2 shows an example flow charts of operations suitably performed by the apparatus of FIG.1.
FIG. 3 and 4 show different embodiments of the apparatus of FIG. 1.
FIG. 5 shows an example flow charts of operations suitably performed by the apparatus of FIG. 1.

### DETAILED DESCRIPTION OF EMBODIMENTS

As earlier discussed, the ROCC can provide automated assistance to the remote expert and/or local technologist. Such automated assistance may be provided in the form of machine learning (ML)-based image analysis performed on medical images immediately upon their acquisition. Such ML-based analyses can provide early detection of imaging artifacts, poorly chosen field-of-view, incidental findings beyond the reason for the imaging examination, and other issues. By way of early detection of such issues, additional or replacement images can be acquired during the imaging examination, thereby eliminating the need for a call-back examination. ML-based detection of incidental findings can also have immense clinical value by providing early detection of treatable medical conditions. The ROCC may utilize other types of ML models, for example ML models applied to bay camera video to detect events occurring in the imaging bay.

However, the ML models used in such ML-based image analysis require training, which is a computationally complex process. A large suite of ML models can be envisioned to provide detection of a wide range of different types of artifacts, non-optimal imaging conditions, incidental findings, and so forth. However, the training of such a suite of ML models can be taxing even for a server computer or network of servers. Moreover, it may be desirable for some types of ML models to be dynamically trained, i.e. to have update training on a regular basis using recently acquired medical images, in order to ensure the ML models are well-tuned for the particularities of images acquired by newer models of medical imaging devices and newer techniques in medical imaging such a use of new types of contrast agent, new imaging sequences, or so forth.

The following discloses leveraging a layered information technology (IT) architecture to better utilize available computing resources and improve ML-based analyses of complex image and audio analyses to extract actionable information from the bay camera video and possibly other video cameras (in-bore, contrast injector, et cetera) and from a microphone in the imaging bay. Such ML analyses are computationally taxing, and can be prone to error if the training dataset is insufficient.

The layered IT architecture includes the sensors, the "edge" devices, the cloud computing layer, and a centralized application layer. The "edge" devices may, for example, be tablet computers or the like used by the local imaging technician to interface with the centralized application layer. The cloud computing layer corresponds, for example, to the hospital's computing IT network, while the centralized application layer is maintained by a vendor of a medical imaging device. In this architecture, the edge devices carry a substantial workload including camera feed analyses, imaging scanner console scraped screen acquisition and analyses, microphone audio analysis, and conversion to actionable events using various ML models. This is problematic since the edge devices (e.g. tablet computers) typically have limited resources such as computational capacity. On the other hand, while the edge devices have limited computational capacity, they are numerous in a large ROCC network, and as recognized herein they can be effectively leveraged in the aggregate to assist in complex computational tasks.

In one aspect, a training orchestrator tracks usage of the edge devices and reallocates the computing power of those devices that are experiencing downtime (e.g. between examinations, during night shifts, et cetera) to ML model training tasks. The orchestrator also coordinates the ML model training tasks, for example storing an index of ML models and allocating training to edge devices utilizing those models, and tracking success/failure ratios of the ML models to allocate or prioritize which models to train. This aspect leverages the edge devices in the aggregate for the secondary task of assisting in ML model training, without adversely impacting their primary task of assisting imaging technicians and/or experts in imaging examinations.

In another aspect, optical character recognition (OCR) and information extraction from the console display feed are divided into mandatory and conditional categories. Mandatory OCR tasks are those that relate to fields that can in general change throughout the imaging examination, so that the OCR needs to be run throughout the scan. Conditional OCR tasks are those that relate to fields that only change at well-defined phases of the examination. An example of conditional OCR tasks are those relating to patient demographics, which is entered only during the exam startup phase and thereafter remain unchanged Conditional OCR tasks are only performed during their relevant exam phase(s), thus reducing computational load on the edge device. This aspect increases the efficiency of the edge devices in performing their primary task of assisting imaging technicians and/or experts in imaging examinations, by removing the unnecessary processing load entailed in OCR'ing text that is static over a significant portion of an imaging examination.

In another aspect, rather than applying a single ML model to extract information, an ensemble of ML models can be applied, and the final result obtained by combining the outputs of the ensemble using a technique such as voting. This aspect improves the effectiveness of the ML models in assisting imaging technicians and/or experts in imaging examinations.

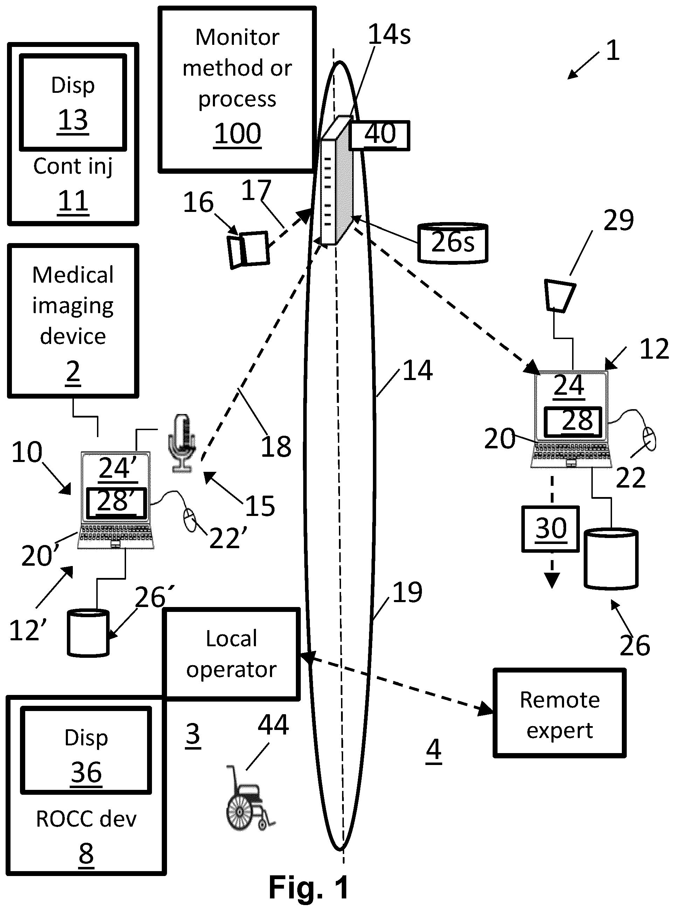

With reference to FIG. 1, a support apparatus **1** for providing assistance from a remote medical imaging expert **RE** (or supertech) to a local technologist operator **LO** is shown. As shown in FIG. 1, the local operator **LO**, who operates a medical imaging device (also referred to as an image acquisition device, imaging device, and so forth) **2**, is located in a medical imaging device bay **3**, and the remote expert **RE** is disposed in a remote service location or center **4.** It should be noted that the "remote expert" **RE** may not necessarily directly operate the medical imaging device **2**, but rather provides assistance to the local operator **LO** in the form of advice, guidance, instructions, or the like. The remote location **4** can be a remote service center, a radiologist's office, a radiology department, and so forth. The remote location **4** may be in the same building as the medical imaging device bay **3** (this may , for example, in the case of a "remote operator or expert" **RE** who is a radiologist tasked with peri-examination image review), but more typically the remote service center **4** and the medical imaging device bay **3** are in different buildings, and indeed may be located in different cities, different countries, and/or different continents. In general, the remote location **4** is remote from the imaging device bay **3** in the sense that the remote expert **RE** cannot directly visually observe the imaging device **2** in the imaging device bay **3** (hence optionally providing a video feed as described further herein).

The image acquisition device **2** can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device **2** may also be a hybrid imaging device such as a PET/CT or SPECT/CT imaging system. While a single image acquisition device **2** is diagrammatically represented in FIG. 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center **4** may provide service to multiple hospitals. The local operator controls the medical imaging device **2** via an imaging device controller **10.** The remote operator is stationed at a remote workstation **12** (or, more generally, an electronic controller **12**).

Some types of imaging modalities may utilize an intravascular contrast agent. For example, MR may utilize a gadolinium-based contrast agent. To provide for contrast-enhanced imaging, a contrast injector **11** is configured to inject the patient with a contrast agent. The contrast injector **11** is a configurable automated contrast injector having a display **13.** The user (usually the imaging technologist) loads a vial or syringe of contrast agent (or two, or more, vials of different contrast agent components) into the contrast injector **11**, and configures the contrast injector **11** by entering contrast injector settings such as flow rates, volumes, time delays, injection time durations, and/or so forth via a user interface (UI) of the contrast injector **11.** The UI may be a touch-sensitive overlay of the display **13**, and/or physical buttons, keypad, and/or so forth. In a variant embodiment, the contrast injector **11** is integrated with the imaging device controller **10** (e.g., via a wired or wireless data connection), and the contrast injector **11** is controlled via the imaging device controller **10**, including displaying the contrast injector settings in a (optionally selectable) window on the display of the imaging device controller **10.** In such an embodiment, the dedicated physical injector display **13** of the contrast injector may optionally be omitted (or, alternatively, the dedicated physical injector display **13** may be retained and the contrast settings displayed at both the dedicated physical injector display **13** and at the imaging device controller **10**). In general, the automated contrast injector **11** can employ any suitable mechanical configuration for delivery of the contrast agent (or agents), such as being a syringe injector, a dual-syringe injector, pump-driven injector, or so forth, and may include hardware for performing advanced functions such as saline dilution of the contrast agent, priming and/or flushing of the contrast injection line with saline, and/or so forth.

As used herein, the term "medical imaging device bay" (and variants thereof) refer to a room containing the medical imaging device **2** and also any adjacent control room containing the medical imaging device controller **10** for controlling the medical imaging device. For example, in reference to an MRI device, the medical imaging device bay **3** can include the radiofrequency (RF) shielded room containing the MRI device **2**, as well as an adjacent control room housing the medical imaging device controller **10**, as understood in the art of MRI devices and procedures. On the other hand, for other imaging modalities such as CT, the imaging device controller **10** may be located in the same room as the imaging device **2**, so that there is no adjacent control room and the medical bay **3** is only the room containing the medical imaging device **2**. In addition, while FIG. 1 shows a single medical imaging device bay **3**, it will be appreciated that the remote service center **4** (and more particularly the remote workstation **12**) is in communication with multiple medical bays via a communication link **14**, which typically comprises the Internet augmented by local area networks at the remote expert **RE** and local operator **LO** ends for electronic data communications. In addition, while FIG. 1 shows a single remote service center **4**, it will be appreciated that the medical imaging device bays **3** is in communication with multiple medical bays via the communication link **14.**

As diagrammatically shown in FIG. 1, in some embodiments, a camera **16** (e.g., a video camera) is arranged to acquire a video stream or feed **17** of a portion of a workspace of the medical imaging device bay **3** that includes at least the area of the imaging device **2** where the local operator **LO** interacts with the patient, and optionally may further include the imaging device controller **10.** While one camera **16** is shown, there may be multiple cameras, e.g. one providing a feed of the imaging bay generally, another providing a video feed of a display **36** of the contrast injector **11**, and/or so forth. In other embodiments, a microphone **15** is arranged to acquire an audio stream or feed **18** of the workspace that includes audio noises occurring within the medical imaging device bay **3** (e.g., verbal instructions by the local operator **LO**, questions from the patient, and so forth). The video stream **17** and/or the audio stream **18** is sent to the remote workstation **12** via the communication link **14**, e.g. as a streaming video feed received via a secure Internet link.

The communication link **14** also provides a natural language communication pathway **19** for verbal and/or textual communication between the local operator and the remote operator. For example, the natural language communication link **19** may be a Voice-Over-Internet-Protocol (VOIP) telephonic connection, an online video chat link, a computerized instant messaging service, or so forth. Alternatively, the natural language communication pathway **19** may be provided by a dedicated communication link that is separate from the communication link **14** providing the data communications **17**, **18**, e.g. the natural language communication pathway **19** may be provided via a landline telephone. In some embodiments, the natural language communication link **19** allows a local operator **LO** to call a selected remote expert **RE.** The call, as used herein, can refer to an audio call (e.g., a telephone call), a video call (e.g., a Skype or Facetime or other screen-sharing program), or an audio-video call. In another example, the natural language communication pathway **19** may be provided via an ROCC device **8**, such as a mobile device (e.g., a tablet computer or a smartphone), or can be a wearable device worn by the local operator **LO**, such as an augmented reality (AR) display device (e.g., AR goggles), a projector device, a heads-up display (HUD) device, etc., each of which having a display device **36.** For example, an "app" can run on the ROCC device **8** (operable by the local operator **LO**) and the remote workstation **12** (operable by the remote expert **RE**) to allow communication (e.g., audio chats, video chats, and so forth) between the local operator and the remote expert. In some examples, when multiple imaging device bays **3** can each include a corresponding ROCC device **8.**

FIG. 1 also shows, in the remote service center **4** including the remote workstation **12**, such as an electronic processing device, a workstation computer, or more generally a computer, which is operatively connected to receive and present the video feed **17** of the medical imaging device bay **3** from the camera **16** and/or to the audio feed **18.** Additionally or alternatively, the remote workstation **12** can be embodied as a server computer or a plurality of server computers, e.g. interconnected to form a server cluster, cloud computing resource, or so forth. The workstation **12** includes typical components, such as an electronic processor **20** (e.g., a microprocessor), at least one user input device (e.g., a mouse, a keyboard, a trackball, and/or the like) **22**, and at least one display device **24** (e.g. an LCD display, plasma display, cathode ray tube display, and/or so forth). In some embodiments, the display device **24** can be a separate component from the workstation **12.** The display device **24** may also comprise two or more display devices. The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation **12**, various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote operator display device **24.** The video feed **17** from the camera **16** can also be displayed on the display device **24**, and the audio feed **18** can be output on the remote workstation **12** via a loudspeaker **29.** In some examples, the audio feed **18** can be an audio component of an audio/video feed (such as, for example, recording as a video cassette recorder (VCR) device would operate).

FIG. 1 shows an illustrative local operator **LO**, and an illustrative remote expert **RE** (e.g., supertech). However, in a Radiology Operations Command Center (ROCC) as contemplated herein, the ROCC provides a staff of supertechs who are available to assist local operators **LO** at different hospitals, radiology labs, or the like. Each remote expert **RE** can operate a corresponding remote workstation **12.** The ROCC may be housed in a single physical location, or may be geographically distributed. For example, in one contemplated implementation, the remote expert **RE** are recruited from across the United States and/or internationally in order to provide a staff of supertechs with a wide range of expertise in various imaging modalities and in various imaging procedures targeting various imaged anatomies. A server computer **14s** can be in communication with the medical imaging bay **3** and the remote service center **4** with one or more non-transitory storage media **26s.** The non-transitory storage media **26s** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer **14s**, various combinations thereof, or so forth. It is to be understood that any reference to a non-transitory medium or media **26s** herein is to be broadly construed as encompassing a single medium or multiple media of the same or different types. Likewise, the server computer **14s** may be embodied as a single electronic processor or as two or more electronic processors. The non-transitory storage media **26s** stores instructions executable by the server computer **14s.**

The medical imaging device controller **10** in the medical imaging device bay **3** also includes similar components as the remote workstation **12** disposed in the remote service center **4.** Except as otherwise indicated herein, features of the medical imaging device controller **10**, which includes a local workstation **12**', disposed in the medical imaging device bay **3** similar to those of the remote workstation **12** disposed in the remote service center **4** have a common reference number followed by a "prime" symbol, and the description of the components of the medical imaging device controller **10** will not be repeated. In particular, the medical imaging device controller **10** is configured to display a GUI **28**' on a display device or controller display **24**' that presents information pertaining to the control of the medical imaging device **2**, such as configuration displays for adjusting configuration settings an alert **30** perceptible at the remote location when the status information on the medical imaging examination satisfies an alert criterion of the imaging device **2**, imaging acquisition monitoring information, presentation of acquired medical images, and so forth. It will be appreciated that the screen mirroring data stream **18** carries the content presented on the display device **24'** of the medical imaging device controller **10.** The communication link **14** allows for screen sharing between the display device **24** in the remote service center **4** and the display device **24**' in the medical imaging device bay **3**. The GUI **28**' includes one or more dialog screens, including, for example, an examination/scan selection dialog screen, a scan settings dialog screen, an acquisition monitoring dialog screen, among others. The GUI **28**' can be included in the video feed **17** and displayed on the remote workstation display **24** at the remote location **4**.

Furthermore, as disclosed herein, the server **14s** performs a method or process **100** for monitoring a medical imaging examination performed using a medical imaging device **2** (i.e., by assisting local operators **LO** of respective medical imaging devices **2** during medical imaging examinations by a remote expert **RE**). The instructions to perform the method **100** are stored in the non-transitory computer readable medium **26s** of the server computer **14s.**

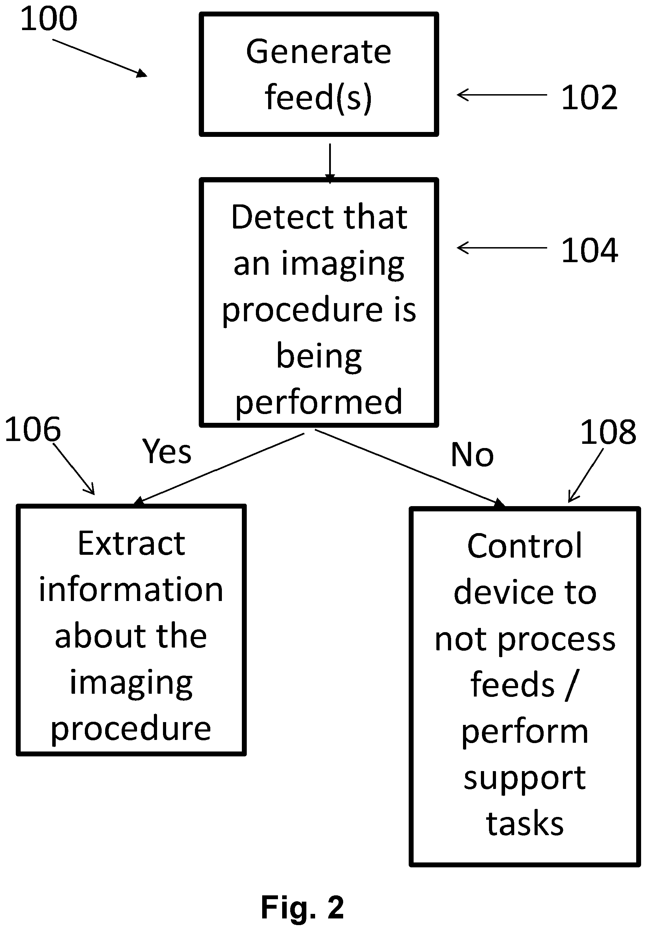

With reference to FIG. 2, and with continuing reference to FIG. 1, an illustrative embodiment of the method **100** in one aspect is diagrammatically shown as a flowchart. In this aspect, the edge devices **11** are leveraged to perform ML training tasks. To begin the method **100**, an imaging examination is commenced by the local operator **LO** using the medical imaging device **2**. An event can occur during the examination which requires assistance from a remote expert **RE.** At an operation **102**, **t**he video feed **17** (acquired by the one or more cameras **16** and/or the audio feed **18** (acquired by the one or more microphones **15**) are routed to the server computer **14s** for analysis. At an operation **104**, the server computer **14s** analyzes the video feeds **17** and/or the audio feeds **18** to detect whether a medical procedure is being performed in the imaging bay **3.** For example, this can be based on whether the patient support is visible and does not have a patient loaded thereon. In another embodiment, the detection **104** can be based on information from the scraped imaging device controller **10**, e.g. if an idle screen is detected then it is determined that the imaging device is not currently in use.

At an operation **106**, in response to the detecting (i.e., the detecting operation **104**) indicating a medical imaging procedure is being performed in the imaging bay **3**, the server computer **14s** is configured to control a local electronic processing device assigned to the imaging bay **3** (i.e., the ROCC device **8**) to process the one or more video feeds **17** or audio feeds **18** to extract and present information about the medical procedure being performed in the imaging bay **3.**

In another disclosed aspect of the operation **106**, the one or more video feeds includes a video feed **17** comprising a scaped controller screen video feed of the medical imaging device controller **10** being used in the medical procedure being performed in the imaging bay **3**. The controlling operation **106** can then include identifying text regions of the scraped controller screen video feed that contain text and categorizing the text regions as quasi-static or dynamic. An optical character recognition (OCR) process can be performed to extract content of the dynamic text regions continuously during the medical procedure being performed in the imaging bay **3**, and another OCR process can be performed to extract content of the quasi-static text regions only at times of the medical procedure being performed in the imaging bay **3** at which content of the quasi-static text regions may change.

These are merely illustrative examples, and should not be construed as limiting.

At an operation **108**, in response to the detecting indicating a medical procedure is not being performed in the imaging bay **3**, the server computer **14s** is configured to control the ROCC device **8** to not process the one or more video feeds **17** and/or audio feeds **18.** For example, the controlling operation **108** can include controlling operation of the at least one camera **16** and/or the microphone **15** to not operate to prevent generation of the video feed **17** and/or the audio feed **18.** Optionally, during these times when the ROCC device **11** is not being used for its primary purpose of supporting an imaging examination, it may be used for the secondary purpose of performing ROCC support tasks, such as training ML models used by the ROCC.

In some embodiments, the controlling operation **108** can include controlling the ROCC device **8** to apply at least one machine learning (ML) component **40** to extract information about medical imaging procedures performed in the imaging bay **3** from images acquired by the medical imaging device (e.g., to detect suboptimal imaging settings, to detect incidental findings, et cetera). For example, a plurality of ML models **40** can be retrieved from the server computer **14s**, and training of at least one of the plurality of ML models **40** can be allocated to the ROCC device **8.** In another example, when multiple ROCC devices **8** are provided in corresponding medical imaging device bays **3**, each ROCC device **8** can be allocated one or more ML models **40** to train. In a further example, a first ML model **40** can be applied to extract a first type of information from the one or more video feeds **17** and/or audio feeds **18.** Multiple second ML models **40** can be applied to extract a second type of information from the one or more video feeds **17** and/or audio feeds **18.** The first and second types of information can then be combined (e.g., by a voting process) to extract the information presented about the medical procedure being performed in the imaging bay **3.**

The results of the training of the ML models **40** can then be received at the server computer **14s** from the ROCC device **8.** The server computer **14s** is then programmed to receive feedback from the ROCC device **8** indicative of performance of each ML model in extracting the information about the medical imaging procedures performed in the imaging bays **3.** The server computer **14s** is further programmed to allocate the ML model training tasks amongst the one or more ML models **40** based on the feedback received from the ROCC device(s) **8** indicative of performance of each ML model **40.**

In some embodiments, the ROCC device **8** is configured to provide a communication interface (i.e., the natural language communication pathway **19**) between the local operator **LO** and the remote expert **RE.**

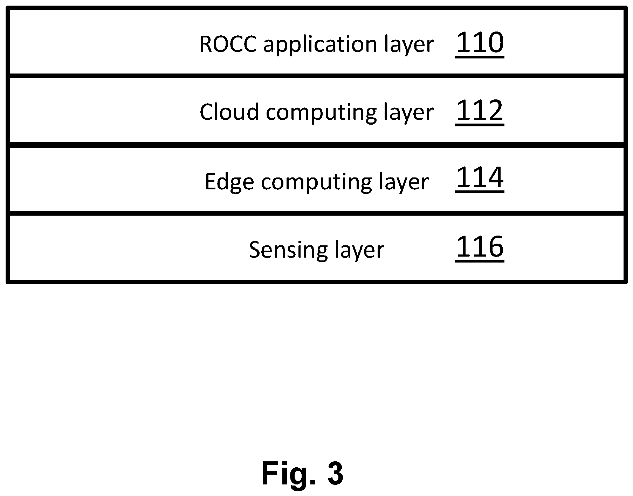

With reference to FIG. 3, a high level representation of the layered computational architecture of the ROCC is shown, including an ROCC application layer **110**, a cloud computing layer **112**, an edge computing layer **114**, and a sensing layer **116.** The bottom most layer is the sensing layer **116** which processes raw data from camera(s) **16** and the controller display. This pre-processed data from the sensing layer **116** is fed to ROCC tablet or other edge device **11** which is part of edge computing layer **114.** Note that each scanner room or imaging bay in general has a dedicated ROCC tablet **11** containing a deep learning (DL), or other ML model (or suite of ML models) suited to its environment (specifically the scanner console and scanner room camera configuration). The edge computing layer **114** processes the data from the sensing layer **116** into events using the ML models. These events are streamed to the cloud account of the cloud computing layer **112** that is specific to the imaging center. The cloud computing layer **112** uses the events to measure workflow metrics and provide status on room, exam etc. This layer **112** also hosts a local repository of the ML models being used at a particular imaging center. The inferences from cloud computing layer **112** are pushed to the ROCC application layer **110** where it is relayed to expert users who can make appropriate imaging examination support decisions. The ROCC application layer **110** also contains the complete list of ML models used across the ROCC platform, to facilitate distributed training and update-training of the ML models.

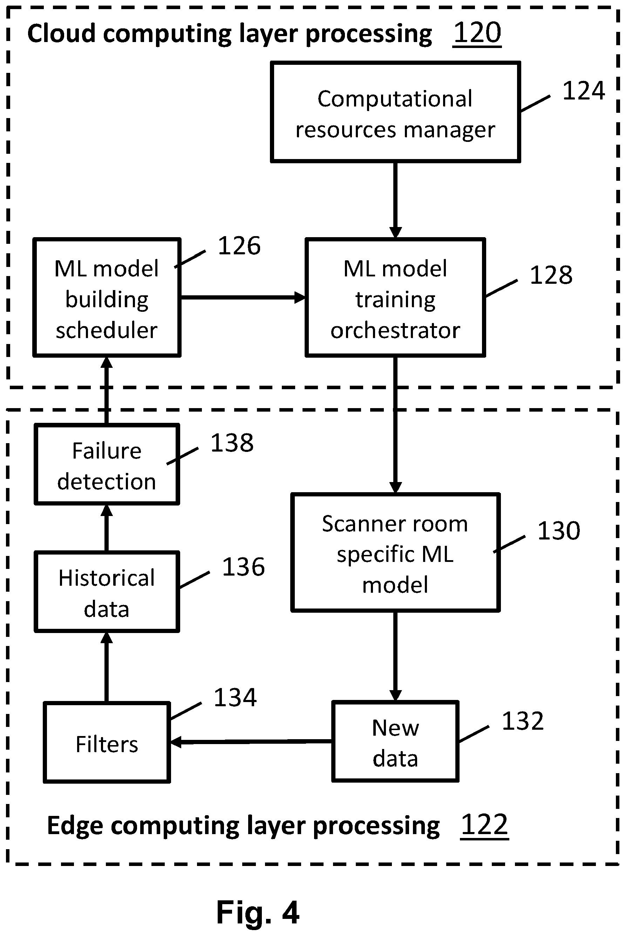

With reference to FIG. 4, an example is shown of leveraging the edge devices during operation **106** of FIG. 2 in accordance with the layered computational ROCC architecture of FIG. 3 to perform secondary ROCC support tasks, such as training or update-training of ML models used by the ROCC. The processing is divided between cloud computing layer processes **120** performed by the cloud computing layer **112**, and processes **122** performed by the edge computing layer **114.** The cloud computing processing **120** include a computational resources manager **124** manages the available computing resources. For example, it may receive the output **102** of the method **100** of FIG. 2 so as to determine which edge devices **11** are available for performing secondary support processing at any given time. A ML model building scheduler **126** schedules ML models to be trained, and a ML model training orchestrator assigns ML training tasks to edge devices based on their availability (from the resources manager **124**) and the ML training tasks to be performed (from the scheduler **126**). The tasks are then performed by the assigned edge devices per operation **106** of FIG. 2.

In the edge computing layer processing **122**, at an operation **130** an edge device **11** uses a trained ML model to perform ROCC examination support tasks. For example, the operation **130** may use trained ML models to identify non-optimal imaging scan settings, incidental findings, or so forth in acquired images so as to provide early warning of such issues. The operation **130** may also use trained ML models applied to frames of the video feed(s) **17** to detect events occurring during the imaging examination. In applying the ML models, the edge device may use model weights, configuration parameters, or so forth that are specific to the imaging scanner **2** or camera **16** being used for the imaging examination. These applications of the ML models in operation **130** produce new data, which may optionally be filtered **134** in various ways to generate (new) historical data **136.** A failure detection model **138** is applied to detect whether the ML model produced correct results. The operation **138** can operate in various ways, such as comparing the ML model output with information that confirms or contradicts that result. As an example, if the ML model outputs a warning that an imaging setting may be incorrect, and the technician adjusts that setting and reacquires the data then this is confirmatory; if on the other hand the technician saves the files without rescanning this is contradictory. The operation **138** thus provides "ground truth" labels as to whether the ML model output is correct or incorrect. Optionally, this feedback may be collected by providing the imaging technician with a short feedback dialog, e.g. asking: "Was this information helpful?" The labeled historical data are then fed back to the ML model building scheduler **126** to perform model update training as appropriate. (For example, if a model has more than some threshold percent contradiction it may be update-trained).

### EXAMPLE

The following describes the support apparatus **1** in more detail. The server computer **14s** can receive data from a sensing layer configured to process the video feeds **17** and the audio feeds **18.** This pre-processed data can be input to the ROCC device **8** (comprising an edge computing layer) that can include one of the ML models **40** configured to process events during the medical imaging examination. The processed data are transmitted to the server computer **14s** and analyzed to measure workflow metrics and provide status updates on the medical imaging examination. The server computer **14s** also stores the ML Models **40.**

The ML models can include a You Only Look Once (YOLO) or another object detection algorithm to generate an event stream. To generate a reliable and accurate event stream, these models **40** need to be trained regularly. The training of these models takes about 200 hours over a dataset containing 5000 images on the ROCC device **8.** In addition, the number of ML models **40** run on ROCC devices **8** is directly proportional to the number of variants in the imaging bay **3.** Given this setup, the training is a repetitive process involving intense computational activities.

The computational resources available in the edge computing layer can be used to perform different services such as ML model **40** training, convert sensed data into events, etc. A computational resource manager tracks the services (e.g., sensing, training, etc.) offered by each resource. The console and camera pipelines are triggered OFF when there is no activity on the console screen and scanner room, respectively. This usually occurs when there is a long-time gap between two consecutive exams and night shifts. In these time periods, the services rendered by the computational sources on the network of ROCC devices **8** are switched to training the ML models **40.** This information is provided to a training orchestrator module implemented in the server computer **14s.**

The training orchestrator module which takes inputs from a ML model building scheduling module and a computational resource manager implemented in the server computer **14s.** The ML model building scheduling module identifies the ML models **40** that require training whereas the computational resource manager identifies the available computational resources within the network of ROCC devices **8** installed at an imaging center. These inputs are used by training orchestrator module to execute the training of the ML models **40.** However, to initiate the training of a ML model **40**, the training orchestrator module compares the current ML model **40** located in a local model repository implemented in the ROCC device **8** with the corresponding ML model **40** in the global model repository implemented in the server computer **14s.** The training is initiated only if both the ML models **40** are identical. For performance improvements, the training job of a ML model **40** is scheduled based on current and target performance metrics (e.g. the number of failures seen in the results obtained from the current deployed ML model **40** and the time since the ML model **40** is last trained). Once the training is complete, the old ML model **40** is replaced with the new trained ML model **40** in both local and global DL model repositories. The computational resource manager chooses the available resources in a way that it balances the workload among the computational resources while reducing the wait time for execution of tasks that are in the queue. To achieve this, it deploys optimization models such as a gradient descent, genetic algorithm. Workload balancing requires pausing ML model training, saving/loading current training states, and restarting ML training without compromising the performance of any tablet to provide the high-priority services e.g. audio-video telepresence, execution of the console and camera algorithms once activity is detected on those input channels, etc.

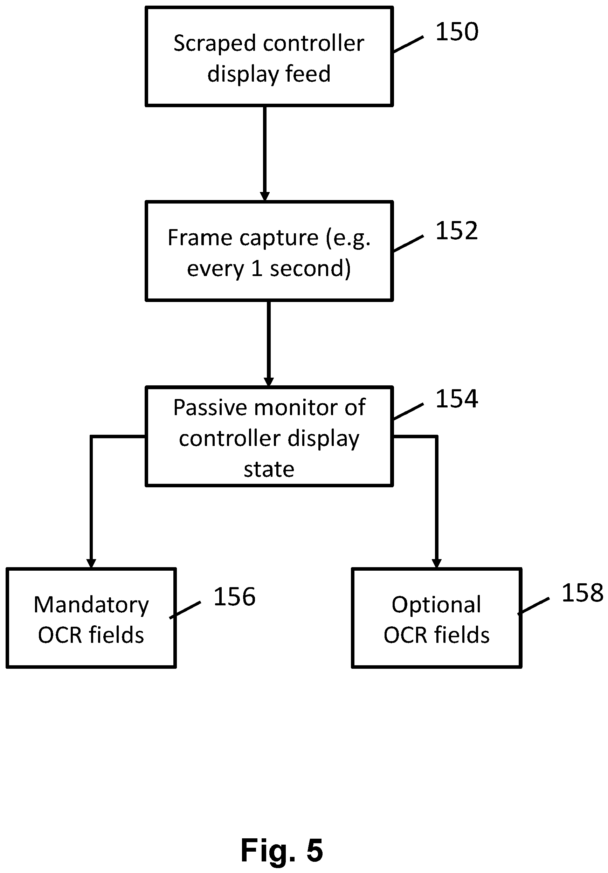

With reference to FIG. 5, in an aspect of the operation **106** of FIG. 2 the OCR of text of the scraped or controller display **24**' may be limited based on content. To this end, the process receives the scraped controller display feed **150**, and captures a frame for processing in an operation **152** at, for example, one frame captured per second. (The capture rate is chosen to balance processing load which is reduced by using a lower capture frequency versus time resolution or responsiveness is increased by using a higher capture frequency). In an operation **154**, the display is monitored to detect and classify text fields. This can for example be done by applying *a priori* known controller display templates, such as templates for the patient intake display, the scan setup display, the acquisition monitoring display, the image review display, and/or so forth. These templates may be specific to the make/model of the medical imaging device. Additionally or alternatively, the current frame can be compared with the last-captured frame to detect changes. Each text region is then classified as a mandatory OCR field **156** or as an optional OCR field **158.** As a specific example, an ROCC console event stream is generated by OCR and passive monitor pipelines. An OCR pipeline is run on multiple fields (>10) on the console screen of the medical imaging device controller **10.** It takes about 1-2 seconds per field to execute the OCR pipeline. This execution may be performed sequentially, and the time taken by OCR pipeline puts a limit on the sensing frequency from the console feed. To address this, the server computer **14s** applies the process of FIG. 4 and thereby classifies all the fields into two categories (Mandatory and Optional) and use OCR on both categories via parallel processing to reduce the computational time. Optional fields are those fields that undergo changes during an exam start but remain same throughout the exam. Running OCR on these fields are conditioned on certain events such as exam start. The remaining fields fall under Mandatory category. Techniques such as multiprocessing, multithreading are used to run OCR in parallel on mandatory and optional fields. To further reduce the computational burden, the passive monitor **154** is introduced. The passive monitor **154** uses fast and simple image analysis (e.g., template matching or comparison with the last-captured frame) to determine if a pop-up window is present in the image or if there is any change in the current image from previous image. The passive monitor triggers relevant OCR pipeline (Mandatory **156** or optional **158** or both, i.e. for different fields of a single captured controller display frame) based on its findings, so that the OCR pipeline is not run unnecessarily.

A YOLO ML model **40** is used to detect a region of interest (ROI) for a given OCR field in an image. Variations in the text background and a font size of these fields translate in the ROI detection by the YOLO model. It is very important to identify the ROI for each OCR field correctly. The current implementation relies on a single YOLO model making it prone to misdetections. To address this, multiple YOLO models (i.e., five) can be used to detect the fields on a given image. Consensus on the detection of the field is derived by using mechanisms such as a voting process.

Unlike a single model implementation, the best ROI is identified by multiple ML models **40.** Different merging strategies such as overlapping ROI or F1-score weighted combination of ROIs can be used to determine the best ROI. This way of using ensemble of the ML models **40** to determine the bounding box makes the ROI detection robust. Running multiple ML models **40** on an image require additional computation time. To overcome this, these ML models **40** are run using parallel processing and trained using a service switching process.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium (**26s**) storing instructions executable by at least one electronic processing device (**8**, **14s**) to perform a method (**100**) of monitoring a medical imaging examination, the method comprising:
receiving one or more video feeds (**17**) of at least an imaging bay (**3**);
detecting, from the one or more video feeds, whether a medical procedure is being performed in the imaging bay;
in response to the detecting indicating a medical procedure is being performed in the imaging bay, controlling a local electronic processing device (**8**) assigned to the imaging bay to process the one or more video feeds to extract and present information about the medical procedure being performed in the imaging bay; and
in response to the detecting indicating a medical procedure is not being performed in the imaging bay, controlling the local electronic processing device to not process the one or more video feeds.

2. The non-transitory computer readable medium (**26s**) of claim 1, wherein the method (**100**) further includes:
receiving an audio feed (**18**) acquired by at least one microphone (**15**) disposed in the imaging bay (**3**);
wherein the controlling of the electronic processing device (**8**) to not process the one or more video feeds further includes controlling operation of the at least one camera or the microphone to not operate to prevent generation of the video feed and the audio feed.

3. The non-transitory computer readable medium (**26s**) of either one of claims 1 and 2, wherein the method (**100**) further includes:
in response to the detecting indicating a medical procedure is not being performed in the imaging bay (**3**), controlling the local electronic processing device (**8**) to perform one or more training tasks for a machine learning (ML) component (**40**).

4. The non-transitory computer readable medium (**26s**) of claim 3, wherein the method (**100**) further includes:
retrieving a plurality of ML models (**40**); and
allocating training of at least one of the plurality of ML models to the local electronic processing device (**8**).

5. The non-transitory computer readable medium (**26s**) of any one of claims 1-4, wherein the one or more video feeds includes a video feed (**17**) comprising a scraped controller screen video feed of a medical imaging device controller (**10**) being used in the medical procedure being performed in the imaging bay, and the controlling of the local electronic processing device (**8**) to process the one or more video feeds to extract and present information about the medical procedure being performed in the imaging bay includes:
identifying text regions of the scraped controller screen video feed that contain text; and
categorizing the text regions as quasi-static or dynamic;
performing optical character recognition (OCR) to extract content of the dynamic text regions continuously during the medical procedure being performed in the imaging bay; and
performing OCR to extract content of the quasi-static text regions only at times of the medical procedure being performed in the imaging bay at which content of the quasi-static text regions may change.

6. The non-transitory computer readable medium (**26s**) of any one of claims 1-5, wherein the controlling of the local electronic processing device (**8**) to process the one or more video feeds (**17**) to extract and present information about the medical procedure being performed in the imaging bay (**3**) includes:
applying a first machine-learning (ML) model (**40**) to extract first information from the one or more video feeds (**17**);
apply a plurality of second ML models to extract second information from the one or more video feeds; and
combining the first and second information to extract the information presented about the medical procedure being performed in the imaging bay.

7. The non-transitory computer readable medium (**26s**) of claim 6, wherein the combining comprises using a voting process.

8. The non-transitory computer readable medium (**26s**) of any one of claims 1-7, wherein the local electronic processing device (**8**) is further programmed to provide a communication interface between a user of the local electronic processing device and a remote expert located remotely from the imaging bay to which the local electronic processing device is assigned.

9. A support apparatus for medical imaging, the support system comprising:
a server computer (**14s**); and
local electronic processing devices (**8**) assigned to respective medical imaging bays (**3**) and programmed to apply machine learning (ML) models (**40**) to video feeds (**17**) received from their respective assigned imaging bays to extract information about medical imaging procedures performed in their respective assigned imaging bays;
wherein the server computer and/or the local electronic processing devices are programmed to determine whether medical imaging procedures are being performed in the respective medical imaging bays; and
wherein the server computer is programmed to perform training of the ML models including allocating ML model training tasks amongst the local electronic processing devices based on whether medical imaging procedures are being performed in the corresponding assigned medical imaging bays and receiving results of the allocated ML model training tasks from the local electronic processing devices.

10. The support apparatus of claim 9, wherein:
the server computer (**14s**) receives feedback from the local electronic processing devices (**8**) indicative of performance of each ML model (**40**) of the ML models in extracting the information about the medical imaging procedures performed in the respective assigned imaging bays (**3**); and
the server computer is further programmed to allocate the ML model training tasks amongst the ML models based on the feedback received from the local electronic processing devices indicative of performance of each ML model.

11. The support apparatus of either one of claims 9 and 10, wherein each local electronic processing device (**8**) is further programmed to provide a communication interface (**19**) between a user of the local electronic processing device and a remote expert located remotely from the imaging bay (**3**) to which the local electronic processing device is assigned.

12. A non-transitory computer readable medium (**26s**) storing instructions executable by at least one electronic processing device (**8**, **14s**) to perform a method (**100**) of monitoring a medical imaging examination, the method comprising:
receiving one or more video feeds (**17**) of at least an imaging bay (**3**);
detecting, from the one or more video feeds, whether a medical procedure is being performed in the imaging bay;
in response to the detecting indicating a medical procedure is being performed in the imaging bay, controlling a local electronic processing device (**8**) assigned to the imaging bay to process the one or more video feeds to extract and present information about the medical procedure being performed in the imaging bay; and
in response to the detecting indicating a medical procedure is not being performed in the imaging bay (**3**), controlling the local electronic processing device (**8**) to perform one or more training tasks for a machine learning (ML) component (**40**).

13. The non-transitory computer readable medium (**26s**) of claim 12, wherein the method (**100**) further includes:
receiving an audio feed (**18**) acquired by at least one microphone (**15**) disposed in the imaging bay (**3**);
wherein the controlling of the electronic processing device (**8**) to not process the one or more video feeds further includes controlling operation of the at least one camera or the microphone to not operate to prevent generation of the video feed and the audio feed.

14. The non-transitory computer readable medium (**26s**) of either one of claims 12 and 13, wherein the method (**100**) further includes:
in response to the detecting indicating a medical procedure is not being performed in the imaging bay, controlling the local electronic processing device to not process the one or more video feeds.

15. The non-transitory computer readable medium (**26s**) of claim 12, wherein the method (**100**) further includes:
retrieving a plurality of ML models (**40**); and
allocating training of at least one of the plurality of ML models to the local electronic processing device (**8**).
